# EUROPEAN PATENT APPLICATION

(11) **EP 1 964 926 A1**
(43) Date of publication of application: **03.09.2008**
(21) Application number: 06834105.6
(22) Date of filing: 06.12.2006
(51) Int. Cl.: C12Q 1/02, C12M 1/34

(54) **METHOD OF MEASURING CELL MOTILITY AND SYSTEM THEREFOR**

(30) Priority: 07.12.2005 JP 2005353580
(71) Applicant: Effector Cell Institute, Inc., Tokyo 153-0041 (JP)
(72) Inventor: NITTA, Nao, EFFECTOR CELL INSTITUTE, INC., Tokyo 1530041 (JP)
(74) Representative: Hackney, Nigel John
(86) International application number: PCT/JP2006/324350
(87) International publication number: WO 2007/066684

(57) **Abstract**

The present invention is intended to provide a technique whereby quantitative data of cellular chemotactic activity can be obtained. Namely, it relates to a method of measuring cell motility whereby the motility state of cells is measured. Living cells are placed in an observation space (100) in such a state as allowing the movements the cells on a plane. A stimulus affecting the cell motility is injected in the observation space (110) and then at least one of the followings is measured: (a) the reaction time required from the injection of the stimulus until the movement start as to each of the cells; and (b) the movement-duration time as to each of the cells that have started the movement.

## Description

### Technical Field

The present invention relates to a technique for evaluating cell motility activity.

### Background Technique

Cellular chemotaxis is a function that a cell detects a concentration gradient of a specific factor, and migrates to a higher concentration thereof. Migration of cells has an important role in various vital processes such as inflammatory response, wound healing, homeostatic circulation of lymphocytes or the like, and tumor metastasis and development. Various cells related to immunity are mainly related to many kinds of inflammatory diseases, such as asthma concerning eosinophil and T_{H}2 cell, articular rheumatism/multiple sclerosis and atherosclerosis concerning monocyte and T_{H}1 cell, myocardial infarction/cerebral infarction and ischemia concerning neutrophils, and in addition, Crohn's disease/psoriasis, transplant rejection, hepatitis, type I diabetes, and chronic obstructive pulmonary disease (Andrew et al, 2005).

In analyzing those vital phenomenon and diseases, it is important to evaluate chemotaxis of related cells. This requires a technique which appropriately quantifies the cell motility. If a method for evaluating the cell motility is specified, which can be used as an index indicating presence or absence of disease, seriousness thereof, and drug effectiveness, and the like, a technique for evaluating the cell motility according to the method is particularly useful for the purpose of diagnosis and drug selection against the diseases. Even more particularly, as against a disease in which the chemotaxis plays a significant role in the process thereof, a technique for appropriately evaluating the cell motility is applicable for other purposes, such as resolving a mechanism of the disease and a drug seeking against the disease.

Boyden chamber method has been used as a conventional technique for a long time for quantifying the cellular chemotaxis (Boyden, 1962). In this method, a filter is used, which is provided with a large number of micropores each having a uniform size, and cell are added on one side thereof and a chemotactic factor is added on the other side. This method utilizes migration of the cells that pass through the filter along the concentration gradient of the chemotactic factor, which is generated in the micropores of the filter. Therefore, according to this method, a degree of chemotaxis can be quantified by counting a rate of the cells that have passed through the filter, but it has been impossible to conduct more complex chemotactic pattern analysis and to directly analyze a state of the cells which are in the course of migrating.

As a method to directly observe the cellular chemotaxis by an optical way, there have been developed under agarose method (Cutler and Munoz, 1974; Nelson et al., 1975; John and Sieber, 1976), Zigmond chamber method (Zigmond, 1977), and Dunn chamber method (Zicha et al., 1991), and these techniques have been utilized for studies. Any of those methods above use a minute planar space formed in the horizontal direction. In the under agarose method, when agarose gel is solidified on a glass surface, a crevice is generated in an interface between the glass and the gel, and this crevice is utilized. The Zigmond method and Dunn method employ a special glassware tool on which steps are formed. Cells are placed in a horizontal microspace, as well as a concentration gradient of chemotactic factor is generated, and thereby it is possible to observe the state of the cells by a microscope, under the condition that the cellular chemotaxis is induced.

Furthermore, as a tool for analyzing the chemotaxis, there has been reported KK-chamber method (Kanegasaki et al.,2003). This method stably forms a concentration gradient of Ligand, and directly observes the state of the cells in there. This method has allowed an induction of cellular chemotaxis with a high reproducibility, further enabling the observation of the cellular state on a two-dimensional plane.

In addition, as a technique for quantifying the cell motility on the two-dimensional plane, there has been reported a technique which acquires tracking data of cells, one by one, from a time-lapse image obtained by the microscope, and analyzes the number of cells, a migrating velocity, and the like (Abraham et al., 2004, Krooshoop et al. , 2003, and Wick et al., 2003).
[Non-patent document 1]
   Abraham VC, Taylor DL, Haskins JR. High content screening applied to large-scale cell biology. Trends Biotechnol., 2004 Jan; 22(1): 15 - 22.
[Non-patent document 2]
   Andrew D Luster, Ronen Alon & Ulrich H von Andrian. Immune cell migration in inflammation: present and future therapeutic targets. Nature Immunol, 2005 December, 6(12), pp.1182 to 1190
[Non-patent document 3]
   Boyden, S., 1962. Chemotactic effect of mixtures of antibody and antigen on polymorphonuclear leukocytes. J. Exp. Med. 115, 453.
[Non-patent document 4]
   Cutler, J.E. and Munoz, J. J., 1974., A simple in vitro method for studies on chemotaxis. Proc. Soc. Exp. Biol. Med. 147, 471.
[Non-patent document 5]
   Kanegasaki S, Nomura Y, Nitta N, Akiyama S, Tamatani T, Goshoh Y, Yoshida T, Sato T, Kikuchi Y. A novel optical assay system for the quantitative measurement of chemotaxis. J Immunol Methods. 2003 Nov; 282(1-2): 1-11.
[Non-patent document 6]
   John, T.J. and Sieber, O. F, Jr., 1976. Chemotactic migration of neutrophils under agarose. Life Sci. 18, 177.
[Non-patent document 7]
   Krooshoop, D. J., Torensma, R, van den Bosch, G. J., Nelissen, J. M., Figdor, C. G., Raymakers, R. A. and Boezeman, J. B., An automated multi well cell track system to study leukocyte migration. J. Immunol. Methods. 2003. Sep; 280(1-2): 89 - 102.
[Non-patent document 8]
   Nelson, F. D., Quie, P. G. and Simmons, R. L., 1975. Chemotaxis under agarose: a new and simple method for measuring chemotaxis and spontaneous migration of human polymorphonuclear leukocytes and monocytes. J. Immunol. 115, 1650.
[Non-patent document 9]
   Wick N, Thurner S, Paiha K, Sedivy R, Vietor I, Huber LA. Quantitative measurement of cell migration using time-lapse videomicroscopy and non-linear system analysis. Histochem Cell Biol. 2003 Jan; 119(1): 15 - 20.
[Non-patent document 10]
   Zigmond S. H., 1977. Ability of polymorphonuclear leukocytes to orient in gradients of chemotactic factors. J. Cell Biol. 75, 606.
[Non-patent document 11]
   Zichia, D., Dunn, G. A. and Brown, G. E., 1991. A new direct viewing chemotaxis chamber. J. Cell Sci. 99, 769.

### Disclosure of the Invention

### Problem to be Solved by the Invention

Diagnosis of various diseases may depend on a qualitative analysis of a symptom shown by a patient. In such a qualitative diagnosis, a tendency of the diagnostic result may vary depending on a doctor who undertakes diagnostic evaluation. It is preferable to prepare a quantitative and objective evaluation standard, so as to figure out a disease state of the patient objectively, and to select a drug type and dosage, a treatment method, and the like, according to a result of the diagnosis. By way of example, when a diagnosis of asthma is conducted, conventionally, there have been performed a qualitative evaluation of the symptoms, such as difficulty of breathing and difficulty in actions i.e., walking/speaking, or using an index such as a peak flow rate and oxygen saturation degree of blood. However, the method has limitations if there is a quantitative evaluation of severity of disease or grouping of diseases. Therefore, it has been a problem how to conduct such quantitative evaluation in diagnosing a disease.

The cellular chemotaxis plays an important role in various diseases including asthma. Therefore, if there is acquired an index which appropriately quantifies the cellular chemotaxis concerning those diseases, it is considered extremely useful for diagnosing disease, resolving mechanism thereof, drug development and evaluation (Andrew et al., 2005). However, in the conventional method developed and employed for the purpose of measuring the cell motility, there has not been a cellular chemotaxis evaluation technique, which is at a practical level to be available for diagnosing diseases such as allergy or asthma.

An object of the present invention is to provide a technique for acquiring quantitative data of the cellular chemotaxis, which is available for evaluating various diseases related to the cellular chemotaxis.

### Means to Solve the Problem

According to the first aspect of the present invention, the cell motility measuring method for measuring a cell motility state, including the steps of,
placing living cells in an observation space in such a manner as movable on a plane,
injecting a stimulus for exerting an effect on the cell motility into the observation space in which the cells are accommodated, and
measuring at least either one of the followings;
(a) a reaction time, as to each of the cells, required from a stimulus injection time point to a time of starting movement, and
(b) a duration time while the movement continues, as to each of the cells that started the movement.

According to another aspect of the present invention, there is provided a cell motility measuring system for measuring a cell motility state, including,
an observation support unit which constitutes an observation space for holding living cells in such a manner as being movable on a plane, and which is capable of injecting into the observation space, a stimulus for exerting an effect on the cell motility,
an imaging device for taking images of the cells within the observation support unit, and
a computer for processing the images acquired by the imaging device, wherein,
the computer is connected to a storage device, a display device, and an input device, and including
a means for storing the images from the imaging device in the storage device and storing time information indicating imaging time points, respectively in association with the images,
a means for accepting of an input of stimulus injection time point, acquiring and storing the time point in the storage device, and
a means for obtaining an evaluation index of the cell motility by analyzing the images being acquired, wherein,
the means for obtaining the evaluation index executes the processing of,
extracting from the images being acquired, a distance between a position of each of the cells shown in the image acquired at each imaging time point, and a position of each of the cells shown in the image acquired at the next imaging time point,
calculating an elapsed time between the imaging time points used for obtaining the distance, and calculating a velocity of each of the cells by dividing the distance by the elapsed time, and
determining whether or not the velocity being calculated for each time point with respect to each of the cells reaches at least a threshold, and calculating a time required from the stimulus injection time point up to the time when reaching at least the threshold, so as to obtain the reaction time.

According to further alternative aspect of the present invention, there is provided a cell motility measuring system for measuring a cell motility state, including,
an observation support unit which constitutes an observation space for holding living cells in such a manner as being movable on a plane, and which is capable of injecting into the observation space, a stimulus for exerting an effect on the cell motility,
an imaging device for taking images of the cells within the observation support unit, and
a computer for processing the images acquired by the imaging device, wherein,
the computer is connected to a storage device, a display device, and an input device, and including,
a means for storing the images from the imaging device in the storage device and storing time information indicating imaging time points, respectively in association with the images,
a means for accepting of an input of stimulus injection time point, acquiring and storing the time point in the storage device, and
a means for obtaining an evaluation index of the cell motility by analyzing the images being acquired, wherein,
the means for obtaining the evaluation index executes the processing of,
extracting from the images being acquired, a distance between a position of each of the cells shown in the image acquired at each imaging time point, and a position of each of the cells shown in the image acquired at the next imaging time point,
calculating an elapsed time between the imaging time points used for obtaining the distance, and calculating a velocity of each of the cells by dividing the distance by the elapsed time, and
determining whether or not the velocity being calculated for each time point with respect to each of the cells reaches at least the threshold, determining whether or not the velocity being calculated for each time point with respect to each of the cells reaches lower than the threshold, and calculating an elapsed time between the time point reaching at least the threshold and the time point reaching lower than the threshold, so as to obtain the duration time.

### Effect of the Invention

According to the present invention, it is possible to acquire by measurement, quantitative data of cellular chemotactic activity, which is available for evaluating various diseases concerning the cellular chemotaxis. By employing this technique, quantitative data of cellular chemotactic activity can be obtained, which is usable for evaluating various diseases concerning the cellular chemotaxis, including inflammatory diseases such as asthma.

### Brief Description of Drawings

FIG. 1 is a schematic configuration diagram of a system for measuring the cellular chemotaxis according to one embodiment of the present invention;
FIG. 2 illustrates a state where a concentration gradient is provided in the observation support unit that is used in one embodiment of the present invention;
FIG. 3 is a flow chart showing a procedure of an image analyzing process according to one embodiment of the present invention;
FIG. 4 is a graph schematically showing an evaluation index according to one embodiment of the present invention;
FIG. 5 shows screen examples of observation results of the eosinophil chemotaxis;
FIG. 6a - FIG. 6d schematically illustrate a state of migratory pathway tracking of eosinophil chemotaxis;
FIG. 7 illustrates histograms showing the temporal change of the cell velocity;
FIG. 8a and FIG. 8b illustrate comparison of eosinophil chemotaxis according to the VD plot;
FIG. 9a and FIG. 9b illustrate the results obtained by analyzing the reaction time;
FIG. 10a and FIG. 10b illustrate the results obtained by analyzing the duration time;
FIG. 11 is a table showing the blood samples used for the experiment;
FIG. 12a and FIG. 12b illustrate the results obtained by analyzing the eosinophil chemotaxis of each of the disease groups according to the VD plot;
FIG. 13a and FIG. 13b illustrate the results obtained by analyzing the reaction time before the time when the cells start movement; and
FIG. 14a and FIG. 14b illustrate the results obtained by analyzing the duration time of the cell movement.

### Denotation of Reference Numerals

100: OBSERVATION SUPPORT UNIT, 110: CELL ACCOMMODATION F SPACE, 180: INJECTOR, 310: OPTICAL SYSTEM, 320: IMAGING DEVICE, 350: COMPUTER, 351: CPU, 352: MEMORY, 353: STORAGE DEVICE, 370: DATA STORAGE AREA, 380: PROGRAM STORAGE AREA

### Best Mode for Carrying out the Invention

Hereinafter, embodiments of the present invention will be explained with reference to the accompanying drawings.

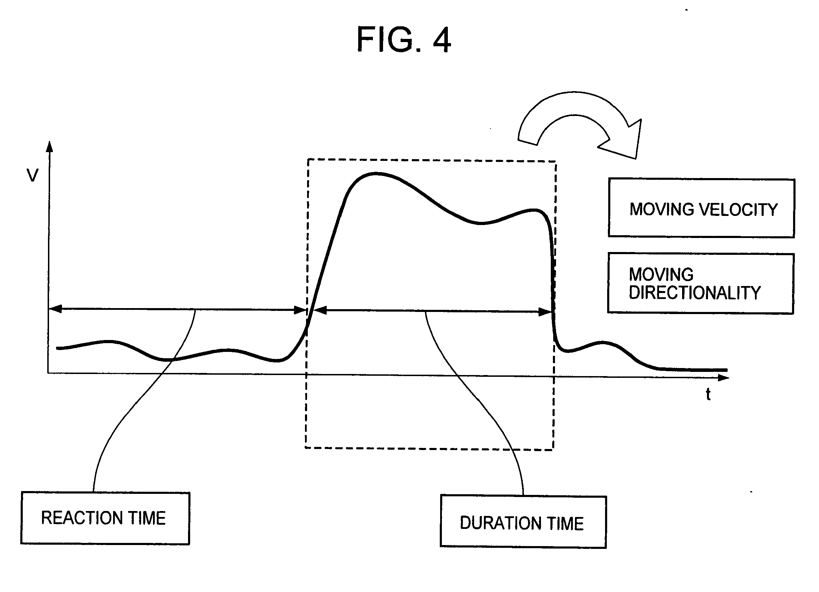

The present embodiment subjects cell motility on a two-dimensional plane to a time-lapse imaging, and analyzes an obtained image. In other words, as schematically illustrated in FIG. 4, it is possible to obtain as an index, a reaction time from a point when a stimulus such as a chemotactic factor is injected against cells, up to a point when the cells start migration individually, or a duration time while the activated cell continues migration, or both of the reaction time and the duration time. The inventors of the present invention have clarified empirically for the first time that these indexes above can be used for diagnosis of diseases such as asthma. A cell motility measuring method to achieve this object will be disclosed in the following.

The embodiments described below are usable in evaluating and diagnosing diseases such as asthma, for instance. Examples described below illustrate an evaluation of eosinophil chemotaxis activity and an analysis example of eosinophil chemotaxis concerning allergy, atopy, and asthma. According to these examples, it is shown that the present embodiments are usable for evaluating the eosinophil chemotaxis, and for diagnosing diseases such as allergy and asthma.

In the examples, "eosinophil" was used as a cell as an analysis object. In addition, "neutrophil", "basophil", "monocyte", "T-lymphocyte", "B-lymphocyte", "macrophage", "mast cell", "dendritic cell", and the like, may be taken as the object cell. Further in the examples, "allergy", "atopy", and "asthma" were taken as object diseases. However, the present invention is not limited to those examples. In addition, "articular rheumatism", "multiple sclerosis", "atherosclerosis", "myocardial infarction", "cerebral infarction", "ischemia", "Crohn's disease", "psoriasis", "transplant rejection", "hepatitis", "type I diabetes", "chronic obstructive pulmonary disease", and the like, may be taken as the example.

In the present embodiment, a cell is chosen and a stimulus is also chosen, thereby achieving a specific support for diagnosis. By way of example, eosinophil is used as the cell and prostaglandin is used as the stimulus, and thereby, a measuring result according to the present embodiment can be utilized for supporting diagnosis concerning allergic disease. In other words, a duration time of the eosinophil as a measurement object is compared with a duration time measured in advance as to the eosinophil of a normal subject. In the case where the duration time of the sample measurement is longer than that of the normal subject, it is possible to output an evaluation that a donor of the eosinophil may suffer from atopic or asthmatic disease. Accordingly, this may support the diagnosis.

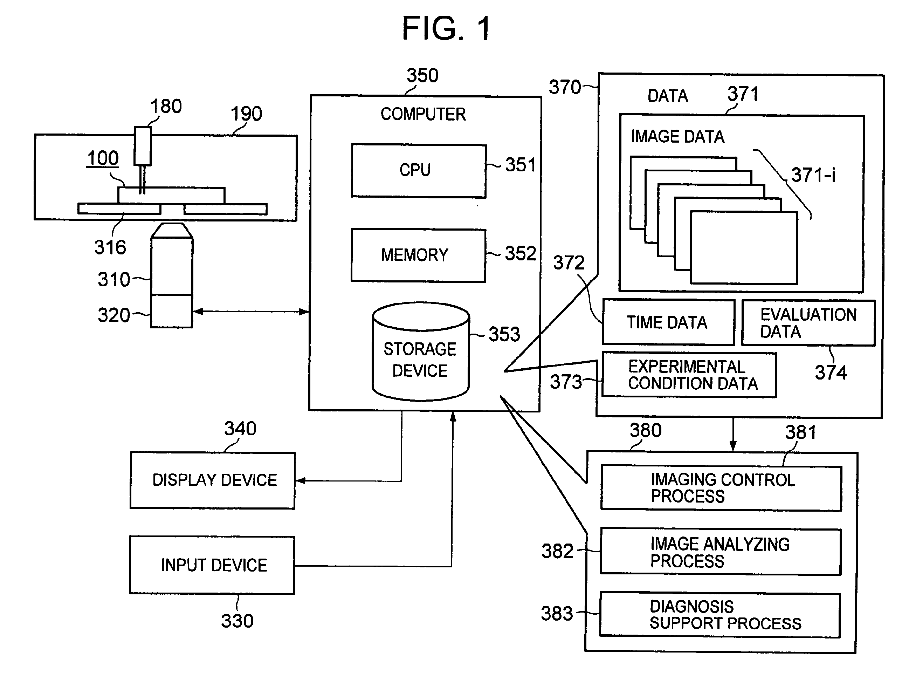

FIG. 1 illustrates one configuration example of the cell motility measuring system as an embodiment of the present invention. The system shown in FIG. 1 is provided with an observation support unit 100 which constitutes an observation space for holding living cells in a such a manner as being movable on a plane, and which is able to inject a stimulus within the observation space for exerting an effect on the movement of the cells, and an imaging device 320 for taking an image of the cells within the observation support unit 100, and a computer 350 for processing the image acquired by the imaging device 320.

The observation support unit 100 has a flat space with a thickness that allows the cells to be movable, and the inside thereof is filled with a culture media, culture solution, or the like, for instance, so as to keep the cells in a viable condition. Specifically, one example is a structure in which multiple grooves are formed on a silicon wafer, and the grooves are utilized as space for accommodating the cells. Since the imaging device 320 takes an image of the observation support unit 100, a surface opposed to the imaging device is formed in such a manner as transparent.

In addition, this observation support unit 100 is provided with a part through which a stimulus is injected in the space where the cells are accommodated. Specifically, a through-hole is provided. The stimulus is injected into the cell accommodation space, by means of an injecting tool 180 such as an injector, for instance.

The stimulus may be injected manually. It is further possible to provide a mechanism of metered-dose injection, which allows an automatic injection based on an injection command from the computer 350. If the injection is conducted automatically, it is convenient when data associated with a clock time of the injection is to be acquired.

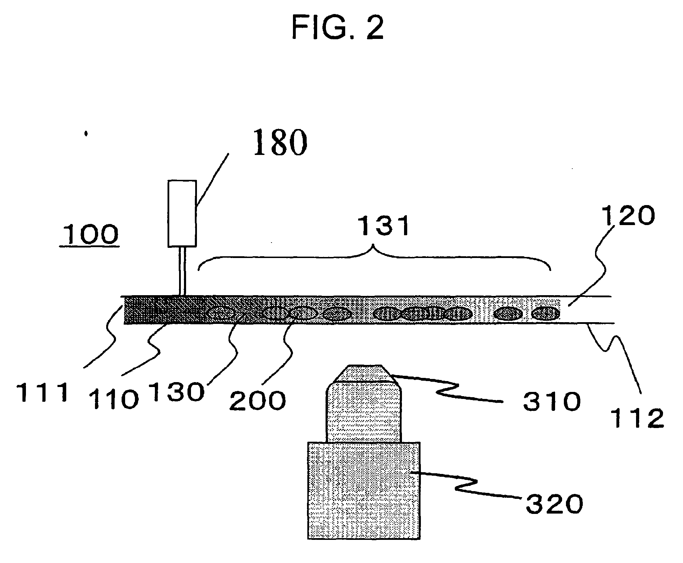

As shown in FIG. 2, in the case of the present embodiment, a concentration gradient of the stimulus used for observing a cellular reaction is formed in the space where a number of cells are placed. Therefore, the observation support unit 100 forms a cell accommodation space 110 as space for accommodating the cells. In order to explain the principle, the cell accommodation space 110 shown in FIG. 1 is schematically illustrated. A specific configuration will be described below.

The cell accommodation space 110 is filled with liquid 120 which is harmless to a cell, and a number of pieces of cells (a group of cells) 200 are placed therein as a target of observation. In this state, a material (referred to as "stimulus") 130 for checking the cellular reaction is injected from one side of the cell accommodation space 110, thereby forming a concentration gradient 131 within the cell accommodation space 110. In this particular example, under circumstances where a solution obtained by dissolving the stimulus 130, e.g., a chemical compound, is injected and the concentration gradient 131 of the specific stimulus 130 is formed, the imaging device 320 such as a digital camera (e.g., CCD camera) takes an image of the state of each of the cells 200, via an optical system 310 constituting a microscope. It is to be noted that in the present embodiment, a photolithographic technique is employed to form more than one piece of (e.g., 12 pieces of) cell accommodation space 110 on silicon ware. Then, the cell motility is measured at each position in the concentration gradient of each cell accommodation space 110. According to this method, it is relatively easy to compare effects of the compound on the cells.

The cell motility toward the stimulus varies depending on the concentration. For example, there are various cellular phenomena as responses from the cells, such as gene expression, morphological change, ejection of biologically active substance, for instance. The cells may indicate as the responses, some feature quantities that can be observed from the outside. The present invention makes use of this property, identifies a feature quantity indicated by a group of cells by analyzing an image of the group of the cells, and acquires information concerning the cell motility, which is associated with the stimulus. In the present invention, the concentration gradient is formed within the same screen. Therefore, trial-and-error works, being time-consuming, are not necessary, such as repeating experiments while varying the concentration. Only one-time experiment allows an observation of a difference of movement, which is caused by a difference in concentration.

FIG. 2 shows an example in which the concentration gradient is formed, but the present invention can be implemented even without providing the concentration gradient. In that case, the stimulus is distributed over the cell accommodation space 110.

The observation support unit 100 is detachably placed in a constant temperature chamber 190 which is thermo-regulated. In addition, the observation support unit 100 is installed on an automatic XY stage 316, and according to a direction from the computer 350, the observation support unit can be displaced on the XY plane. This displacement allows a setting of observation area. The automatic XY stage 316 is provided with a driving mechanism, not illustrated, and performs XY displacement according to an operation command from the computer 350.

The imaging device 320 is, for example, a digital camera equipped with a CCD element, and it acquires an image of the cells and transfers the data to the computer 350. This imaging device 320 takes images at regular intervals, according to a direction from the computer 350. In other words, the imaging device performs a time-lapse imaging.

The computer 350 incorporates a storage device 353, as well as being connected to the display device 340 and the input device 330. In addition, the computer 350 includes a CPU 351 which constitutes a specific means for executing various processing in cooperation with a program so as to process various information, and a memory 352 for loading the program 380 to be executed by the CPU 351, and for storing data 370.

The storage device 353 stores the data 370, including image data 371 transferred from the imaging device 320, time data 372, such as a stimulus injection time point, and an imaging time indicating an imaging time point, experimental condition data 373, and evaluation data 374. The time data indicating each imaging time point is recorded respectively in association with the images having been taken. By way of example, the time data is stored, linking with an image identifier.

The computer 350 includes a control regulation for allowing the imaging device 320 to take an image, a means for storing the image from the imaging device 320 in the storage device 353, and for storing time information indicating the imaging time point in association with the image, a means for accepting an input of the stimulus injection time point, and acquiring and store the time point in the storage device 353, a means for analyzing the image being obtained so as to obtain an evaluation index concerning the cell motility, and a means for supporting diagnosis of diseases by using the evaluation index. The CPU 351 executes the program 380 shown in FIG. 1 to implement the means described above. The program 380 being stored includes an imaging control process 381, an image analyzing process 382, a diagnosis support process 383, and the like. It should be understood here that the programs being stored are not limited to those described above.

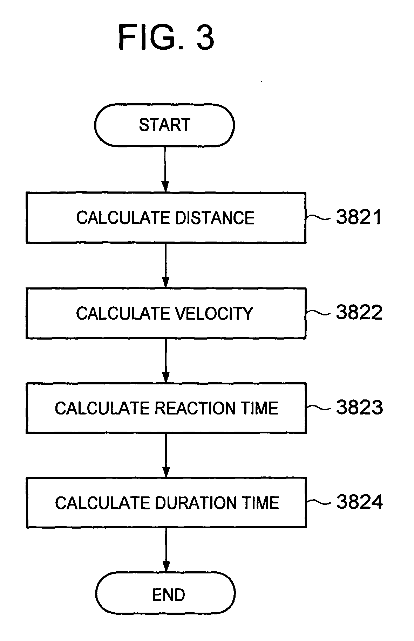

As shown in FIG. 3, the means for obtaining the evaluation index executes a distance calculation process 3821, a velocity calculation process 3822, a reaction time calculation process 3823, and a duration time calculation process 3824. The distance calculation process 3821 extracts a distance from an acquired image, between a position of each of the cells shown in the image obtained at each imaging time point, and a position of each of the cells shown in the image obtained at the subsequent time point. The velocity calculation process 3822 calculates an elapsed time between the imaging time points used for obtaining the distance, and calculates a velocity of each cell by dividing the distance by the elapsed time. The reaction time calculation process 3823 determines whether or not the velocity calculated at each time point as to each cell becomes equal to or higher than a threshold, and upon reaching equal to or higher than the threshold, a time required from the stimulus injection time point up to the time point reaching at least the threshold is calculated, and thereby a reaction time is obtained. The duration time calculation process 3824 determines whether or not the velocity being calculated at each time point as to each cell reaches equal to or higher the threshold, determines whether or not the velocity being calculated becomes lower than a predetermined threshold, and then calculates an elapsed time between the time point reaching equal to and higher than the threshold and the time point reaching lower than the threshold, and thereby a duration time is obtained. The reaction time and the duration time being obtained are stored in the storage device 353 as the evaluation data 374, together with other data serving as the evaluation index.

In the present embodiment, there is shown a configuration that the image analyzing process 382 and the diagnosis support process 383 are performed by the same computer 350 that performs the imaging control process 381. However, the configuration is not limited to this example. By way of example, it is further possible to configure such that the image analyzing process 382 and the diagnosis support process 383 are respectively performed by different computers.

Next, a procedure for measuring the cell motility according to the present embodiment will be explained.

Firstly, cells, for example, eosinophils are injected as a target for analysis, into the cell accommodation space 110 of the observation support unit 100. In this state, a stimulus serving as a stimulating factor such as chemotactic factor, for example, prostaglandin (PGD₂) is injected. This injection can be conducted automatically or manually. On this occasion, the time data is recorded. Specifically, the injection time point is inputted into the computer via the input device 330. By way of example, the display device 340 displays an image including an injection time input area, and when the area is clicked by the input device 330, the imaging control process 381 established by the CPU 351 acquires the input time point and stores it as the time data 372 in the storage device 353. This time is assumed as the injection time point.

The imaging control process 381 subjects the group of cells as the analysis target to the time-lapse imaging, by using the microscope 310 and the imaging device 320. In other words, at predetermined timing, one of the cell accommodation space pieces is subjected to the imaging by the imaging device 320, more than once. Specifically, since the observation support unit 100 is provided with multiple pieces, for example twelve pieces, of the cell accommodation space 110, the imaging is performed sequentially at established time intervals, e.g., every one minute, every five minutes, or the like, while shifting among these pieces of space. The images taken are stored in a form of image data 371 in the data area 370 of the storage device 353.

It is further possible to start the time-lapse imaging before injecting the stimulus. The imaging control process 381 performs processing automatically or manually, to record the followings; a time point when the imaging is started, and time-lapse imaging intervals or a time point when each time-lapse picture is taken.

Next, there will be explained a quantification of the cell motility, i.e., a procedure for obtaining an index according to the image analyzing process.

The CPU 351 identifies the cells one by one from the image data 371 stored in the data area 370 of the storage device 353, and accordingly, determines whether or not individual cells migrated at each time point. Specifically, the CPU 351 extracts from the image being acquired, a distance between a position of each of the cells shown in the image acquired at each imaging time point and a position of each of the cells shown in the image acquired at the next time point (step 3821). A method for acquiring a cell migratory pathway may be performed automatically according to the image processing, or according to a visual judgment by humans. Alternatively, the combination thereof may also be available. If the cell migratory pathway is acquired automatically, a cell image is recognized at a time point and simultaneously a position of the cell image in the screen is identified. At the next time point, another cell image is recognized and a cell image existing at the location being the closest to the previously identified position is determined as the cell image recognized at the previous time point, and this position is specified. A difference between both of the positions above is obtained, and a distance therebetween is calculated.

Next, in judging whether or not the cell has migrated, a migration velocity of the cell is calculated based on the positional information of the cell at each point of time, and when the migration velocity is determined to be exceeding a predetermined threshold, it is determined that the cell has migrated. Therefore, a speed of the migration is calculated. In other words, the CPU 351 calculates an elapsed time between the imaging time points for obtaining the distance, and the distance is divided by the elapsed time to calculate the speed of each cell (step 3822).

Next, it is judged whether or not the speed calculated at each time point as to each cell reaches the threshold or higher, then, a time required from the injection of the stimulus to the time point reaching the threshold or higher is calculated, and the reaction time is obtained (step 3823). Here, the speed is obtained, but it is further possible to obtain a velocity including a migrating direction. Positions of the cell in the images respectively at two time points within the time-lapse are acquired, and a value obtained by dividing a distance therebetween by the interval between the photographing time points can be used as the cell migration velocity.

It is to be noted that the cell migration velocity at each time point may include an error caused by fluctuations or the like in cellular position coordinates at each time point. In order to avoid the situation that the error above causes another error in recognizing the cell migration, it is further possible to use, as the cell velocity for judging whether or not cell migration occurred, a moving average value or an exponential moving average, in addition to an average velocity obtained by dividing the migration distance by the time interval. The moving average value or the exponential moving average is obtained from time-series information acquired by a times-series variation of the average velocity.

In the present embodiment, not only the reaction time but also a duration time is calculated. In other words, the CPU 351 determines whether the calculated speed as to each cell has reached the threshold or higher, and further determines whether the calculated speed has reached lower than the threshold, and obtains an elapsed time between the time when the calculated speed reached the threshold or higher and the time when the calculated speed reached lower than the predetermined threshold, and thereby the duration time is obtained (step 3824).

Here, in setting the threshold, a preferable value is appropriately selected and used, which is suitable for a type of the cell, experimental conditions, a purpose of the measurement, and the like. This value is stored together with the experimental condition data 373, and the data is read out into the memory 352 when the image analyzing process is performed. Then, the CPU 351 uses the threshold in judging the reaction time and the duration time. In the image analyzing process according to the present embodiment, the CPU 351 acquires temporal change of the cell migration velocity from the individual cell migratory pathway, and obtains the response time until the cell starts migration, and the migration duration time until the already-migrating cell stops the migration.

Here, the stimulus injection time point is used as the start point of the time concerning the response time, and the time when the cell started migration is used as the start point of the time concerning the migration duration time. The response time and the duration time are obtained with respect to each cell, one by one. Therefore, if there are thirty cells in the screen, for instance, the information can be obtained as to each of the thirty cells.

The CPU 351 acquires numerical data that is useful for diagnosis or the like, in addition to the information of the response time and the migration duration time. For example, a rate of the cells that started migration at a time point after a lapse of a certain time from the stimulus injection is used. In addition, the % of the cells which continues migration at a time point after a lapse of a certain time from the migration start is also used. In some cases, it is not possible to obtain the information during the measurement due to the following reasons, e.g., the tracking is stopped in mid-course, the migration is not started within the measuring time, and the like. Considering such situations as described above, in order to acquire a temporal change of the rate of the migration-started cell and a temporal change of the rate of the still-migrating cell, a cell from which data was not successfully acquired is treated as censored data, and the Kaplan-Meier estimator, for instance, may be used for acquiring the rate of the migration-started cell and the rate of the still migrating cell in association with each time point. Any of those data items are stored in the data area 370 of the storage device 350 as evaluation data 373.

In addition, the CPU 351 acquires in advance the aforementioned various parameters, including the response time and the duration time concerning a normal subject, and stores such information in the storage device 353.

The use of these evaluation indexes allows a diagnosis support according to the diagnosis support process 383.

### EXAMPLES

Next, an example of the present invention will be explained.

### (Preparation of reagent and cells)

RPMI 1640 Medium HEPES Modification (RPMI-HEPES), Fetal Bovine Serum (FBS) were purchased from Sigma-Aldrich Co.(St. Louis, MO). Dulbecco's Phosphate-Buffered Salines (PBS) was purchased from Invitrogen Japan K. K.(Tokyo, Japan). Recombinant Human MIP-1 alpha (CCL3; Macrophage Inflammatory Protein-1 alpha), Recombinant Human RANTES (CCL5; Regulation upon Activation Normal T cell Express Sequence), and Recombinant Human SDF-1 alpha (CXCL12; Stromal-Cell Derived Factor-1) were purchased from PeproTech Inc. (Rocky Hill, NJ). Other reagents being used were purchased from Wako Pure Chemical Industries, Ltd., Osaka, Japan.

Blood samples were obtained from patients and normal subjects, after a confirmation of informed consent. The blood samples were subjected to haemolytic reaction by water treatment and centrifugal separation, repeated twice, and thereafter a magnetic cell separation method was conducted so as to separate eosinophil therefrom. MACS (Miltenyi Biotec GmbH, Germany) was used for the magnetic cell separation, and the separation was performed by the negative selection method using anti-human CD3, CD14, CD16, and CD19 antibody-labeled paramagnetic microbeads. The magnetic cell separation method was conducted pursuant to the manual of Miltenyi Biotec GmbH. The cells obtained were washed with PBS, and thereafter suspended in RPMI-HEPES culture medium containing 1% FCS at the concentration of 5 × 10⁶ cells/ml, and used for the subsequent experiment.

### (Cellular chemotaxis measurement)

Kaken Geneqs Inc, Chiba, Japan produced the main body of cell motility measuring system and its holder (observation support unit). A silicon tip produced by Yamatake Corporation, Tokyo, Japan was used, and software made by Torii system Co., Ltd., was used for activating the system. Structural setting of the system and the measuring method were carried out according to the aforementioned procedure.

The system used in the present embodiment has the same configuration as shown in FIG. 1. It is the same system as shown in Kanegasaki S, Nomura Y, Nitta N, Akiyama S, Tamatani T, Goshoh Y, Yoshida T, Sato T, Kikuchi Y., "A novel optical assay system for the quantitative measurement of chemotaxis" J Immunol Methods., 2003, Nov; 282(1-2): 1-11.

In the measurement, the observation support unit of the system was filled up with RPMI-HEPES medium containing 1% FCS, and maintained at 37 °C to have the cells and the chemotactic factor being injected, and thereafter, the state of the cells was subjected to the time-lapse imaging. The concentration of the chemotactic factors used for the examination was as the following; CCL3 (MIP-1α) and CCL5 (RANTES) were 5 µM, CXCL12 (SDF-1), N-formyl-methionyl-leucyl-phenylalanine (fMLP), and Prostaglandin D₂ (PGD₂) were 1 µM. Each of the solutions, 1 µl each, was added to the observation support unit 100, and a concentration gradient was formed. Imaging was performed at time-lapse intervals of one minute, and twelve samples at the maximum per one holder were subjected to the imaging in parallel.

### (Cell motility tracking)

The aforementioned program was used for the identification and tracking of the cell. Specifically, dedicated software developed by using C++ Builder 6 (Borland, Cupertino, CA) was employed. With this software, when a user highlighted an area in the image as an analysis target,the cells in the area were identified and tracking was executed. Automatic cell tracking was performed according to the following procedure; a positional change in the time-lapse image was corrected, and then a position of the cell being the closest in the adjacent time-frame photo was sequentially designated and stored. Since an error might be included in the automatic cell tracking, the migratory pathway of the cell being obtained was appropriately corrected based on a visual judgment by a human. Visual designation of the cell pathway had a mechanism as the following; by the present software, the user selected a cell to be analyzed from the time-lapse images, and clicked the position of the cell by the input device, e.g., a mouse, sequentially one frame by one frame, whereby time-series coordinate data of the cell was acquired and stored. According to the obtained migratory pathway information of the cells, one by one, time-series data such as the position, velocity, and directionality of the cells was acquired. In order to avoid exerting an effect of artificial bias on the data, a third person, who was not informed of the details of the experiment, conducted the cell identification and tracking works by using the present software.

### (Statistical analysis)

As to the following analysis, software R (R Development Core Team, 2005) was used for statistical processing calculation and data visualization. VD plot (Velocity-Direction plot) was obtained by calculating median values, as to the values of the time-series data of the velocity and directionality of individual cells, plotting the median value of velocity on the vertical axis and the median value of directionality on the horizontal axis. A gate was set on the graph surface, indicating an index of the cellular chemotaxis, and a rate of the cells included in the gate was obtained. Mann-Whitney U-test was employed for a difference test concerning the rate of the cells.

In the calculation of Chemotactic Response Time (CRT) and of Chemotaxis Lifetime (CLT) of the cell, an Exponential Moving Average (EMA) was calculated based on time-series data of the cell migration velocity, and the cellular chemotaxis was defined by the state where the value of EMA exceeded a certain threshold. In other words, CRT was a time period from start of the measurement until the time when the EMA firstly exceeded the threshold, and an elapsed time from the time when EMA firstly exceeded the threshold until it fell below the threshold was CLT. The calculation of CLT was carried out for the cells which started moving within the measuring time. In the CRT analysis, the cell migration start was set as an index of cellular activation, and according to the CRT of individual cells, a temporal change of the activated cell rate (ACR) in the group of cells was calculated. Similarly, in the CLT analysis, the temporal change of the still-moving cell rate (Running Cell Rate: RCR) was calculated from the CLT values. The cell as to which showed neither the migration start nor the migration stop within the cell tracking time, was treated as censored data. Kaplan-Meier estimator was used for the ACR and the RCR in each time point, and a value of complementary log-log based confidence intervals were used (Venables and Ripley, 1999). As for the ACR and RCR at a specific time, t-test was employed for testing a difference among groups.

### (Eosinophil chemotaxis observation and migratory pathway analysis)

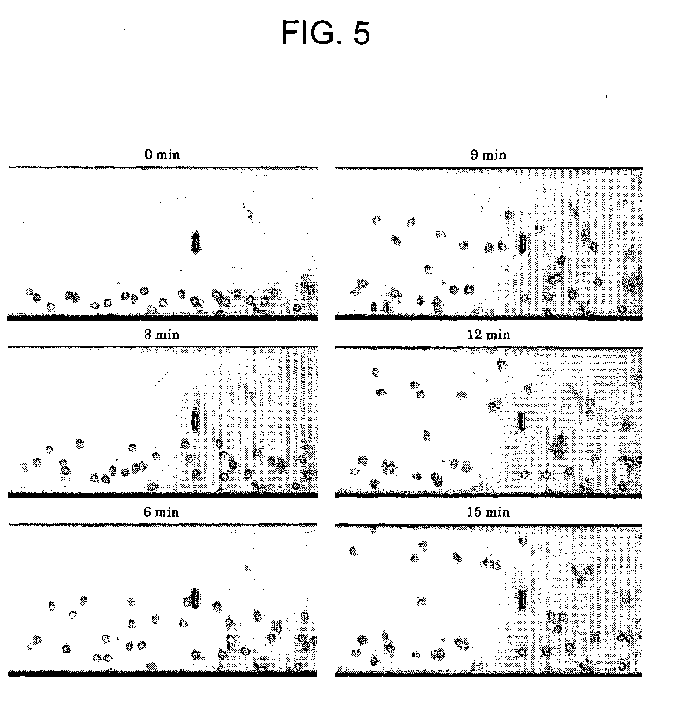

FIG. 5 illustrates images of the state of eosinophils obtained from a normal subject, which are migrating toward the concentration gradient of Prostaglandin D₂ (PGD2), the image being taken by the present system. The concentration gradient of PGD₂ was formed from the upper side directing to the lower side, and it was observed that the eosinophils moved upward the terrace, with the passage of time.

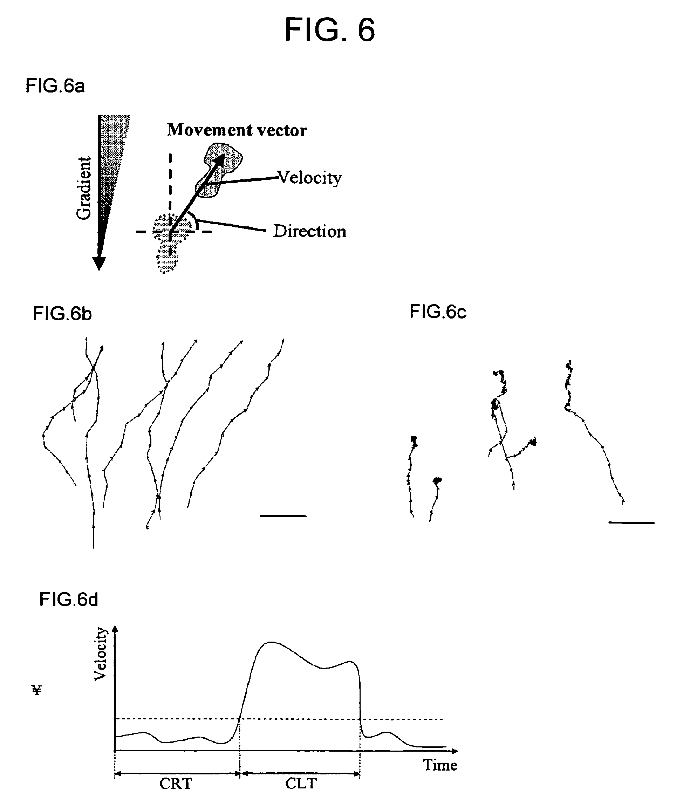

In order to analyze the movement of eosinophils, migratory pathways of the cells were acquired one by one from the time-lapse image. The imaging was performed by time-lapse at every one minute, positional information of each cell at every one minute was obtained from the cell tracking result. By calculating relative values of coordinates of each cell based on two photographs being successive, it is possible to obtain, as to each cell, a migration vector for one minute at each time point. A migration velocity for one minute can be obtained according to the length of the vector, and a specific property of directionality of the movement (direction) against the concentration gradient can be known according to the direction of the migration vector with respect to the concentration gradient direction (FIG. 6a).

The movement directionality was defined by an angle made by a line set in the right angle direction with respect to the concentration gradient direction, and the vector representing the moving direction. In other words, when the cell migrates toward an area of higher concentration along the concentration gradient, the movement directionality indicates +π/2, whereas when the cell migrates toward an area of lower concentration, the movement directionality indicates -π/2. When the cell migrates at right angles to the concentration gradient, the movement directionality indicates zero. By performing the procedure above for the cells one by one with the passage of time, it is possible to obtain a time series variation of the velocity and the movement directionality as to each cell. In the following experiment, migratory pathways of individual cells were acquired from the time-lapse image, and time series variation of the velocity and the movement directionality property obtained therefrom were calculated, and then such time series variation was used in various analyses.

In the eosinophil migration experiment using PGD₂, the cells were divided into two groups, one started migration, and the other did not start migration (data not shown). When the migratory pathways were observed as to the cells that started the migration, there existed a cell that ran through the observation area of the device to the outside (FIG. 6b), and a cell that came to a halt after it migrated for a while (FIG. 6c). It is to be noted that bars shown on the right-lower parts of FIG. 6b and FIG. 6c indicate 50 µm. In this example, it was defined whether or not there was a movement based on a certain threshold, and according to the time series variation of the cell velocity, Chemotactic Response Time (CRT) and Chemotaxis Lifetime (CLT) were acquired as to the cell, and they were set as objects for analysis (FIG. 6d).

In order to study pattern characteristics of the aforementioned movement, the Chemotactic Response Time (CRT) and the Chemotaxis Lifetime (CLT) of the cell were analyzed in the following analysis, according to the time series variation of the cell velocity. In measuring the CRT and CLT of each cell, the time-series data of the velocity was acquired from the cell migratory pathway to calculate the Exponential Moving Average (EMA), and it was defined that when the EMA exceeded a certain threshold, the cell migrated. A position of the cell at each time point was obtained by designating the cells one by one by visual observation, and therefore the position was not necessarily identified uniquely as to a cell that migrated with morphological change. In other words, the position of the cell, and the moving velocity and directionality of the cell calculated from the position might include an inevitable noise. The EMA was employed in the present example, in order to avoid erroneous recognition of the migration start and the like, due to this noise.

### (Eosinophil chemotactic experiment toward various chemotactic factors)

By using the system, an eosinophil chemotactic experiment toward various chemotactic factors was conducted, the eosinophils being obtained from the four blood samples of normal subjects. In the example here, the experiment was conducted under the condition of concentration gradient made by Prostaglandin D₂ (PGD₂), N-Formyl-Met-Leu-Phe (FMLP), CCL3 (MIP-1α), CCL5 (RANTES), and CXCL12 (SDF-1), and under the condition without any compound (N.C.). Here, as for CXCL-12, CXCR4 as a receptor of CXCL-12 hardly appeared on the cell surface of the eosinophil from the peripheral blood and in the blood just after being drawn. Since it was known that the amount of CXCR4 gradually increased when the cell was incubated at 37 °C (Nagase et al. , 2000), the chemotactic experiment with CXCL-12 was conducted after a lapse of eight hours from the time when the eosinophil was separated from the peripheral blood. Concentrations of the various chemotactic factors were experimentally selected, which enabled the most reliable observation of the cellular chemotaxis (data not shown).

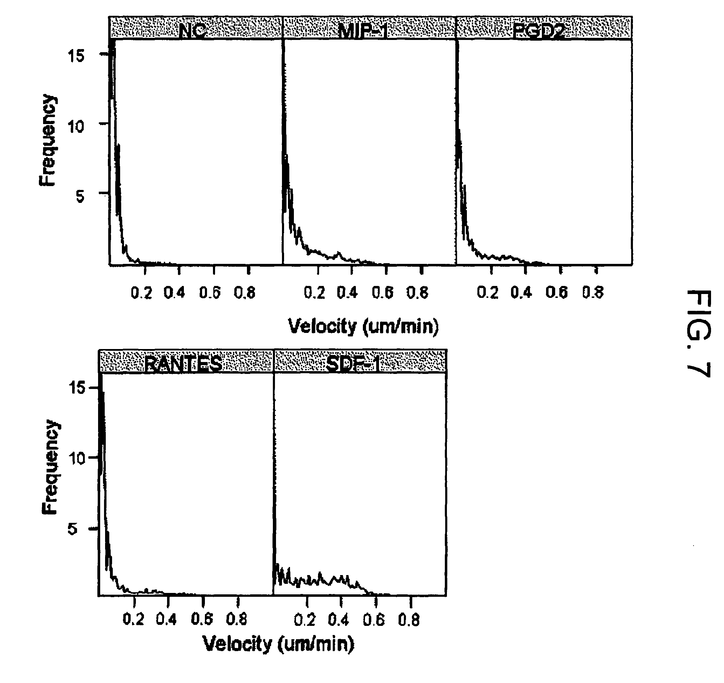

Individual eosinophils were tracked under each of the conditions, and time-series variation information of the velocity and the directionality was acquired based on the obtained pathways. FIG. 7 illustrates histograms of the respective conditions, showing the cell movement velocity as to one of the four normal subject samples. Here, the velocity is represented by the horizontal axis, and the frequency is represented by the vertical axis. According to the data being shown, it was found that under the condition of none treated control (N.C.), total motility of the cells was low, even though a part thereof moved to some extent, and the migration velocity hardly exceeded 0.15 µm/min. Therefore, under the condition of this experiment, it was appropriate to use 0.15 µm/min as threshold for deciding presence or absence of the movement.

### (Analysis of cellular chemotaxis according to VD plot)

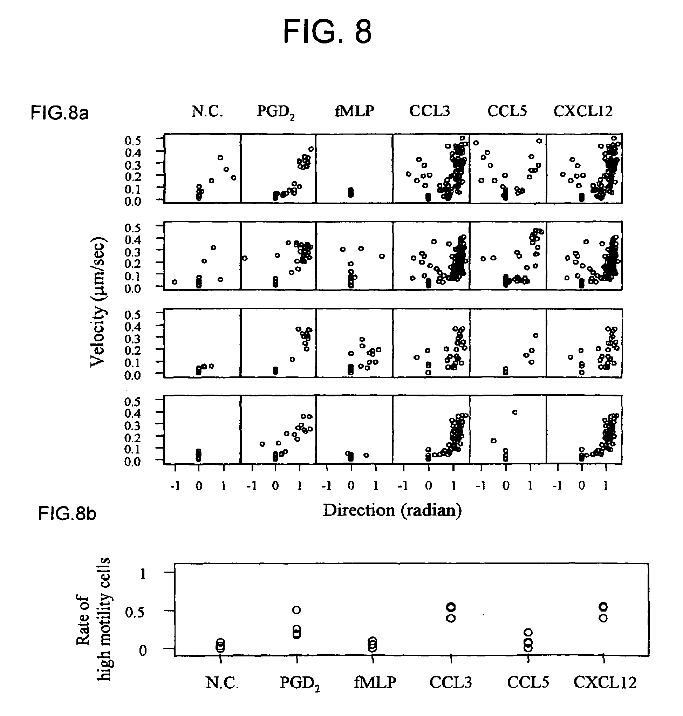

In order to quantitatively analyze the eosinophil chemotaxis toward various chemotactic factors, the cells were tracked and the pathway information was quantitatively analyzed. FIG. 8a shows a diagram (VD plot), obtained by plotting the velocity and the direction of the cellular migration as to individual cells. The VD plot was obtained by calculating a median value (Median) of the velocity and that of the direction based on the tracking data of the cells, and plotting the data by setting the velocity and direction on the vertical axis and the horizontal axis, respectively. Here, it was found that the velocity and the direction of the cells remained around zero and there was almost no movement under the control without any chemotactic factor being added (N.C.), whereas in the case of PGD₂, CCL3, and CXCL12, there were many cells that were plotted in the upper-right area, showing a specific migration in the concentration gradient direction. FIG. 8b illustrates a rate of cells that were detected as having a high specific motility in the concentration gradient direction in the VD plot. According to this figure, it was found that PGD₂, CCL3, and CXCL12 indicated the high motility, but as for fMLP and CCL5, there were little difference from the control, in the analysis by the VD plot.

### (Analysis of response time toward chemotactic factor)

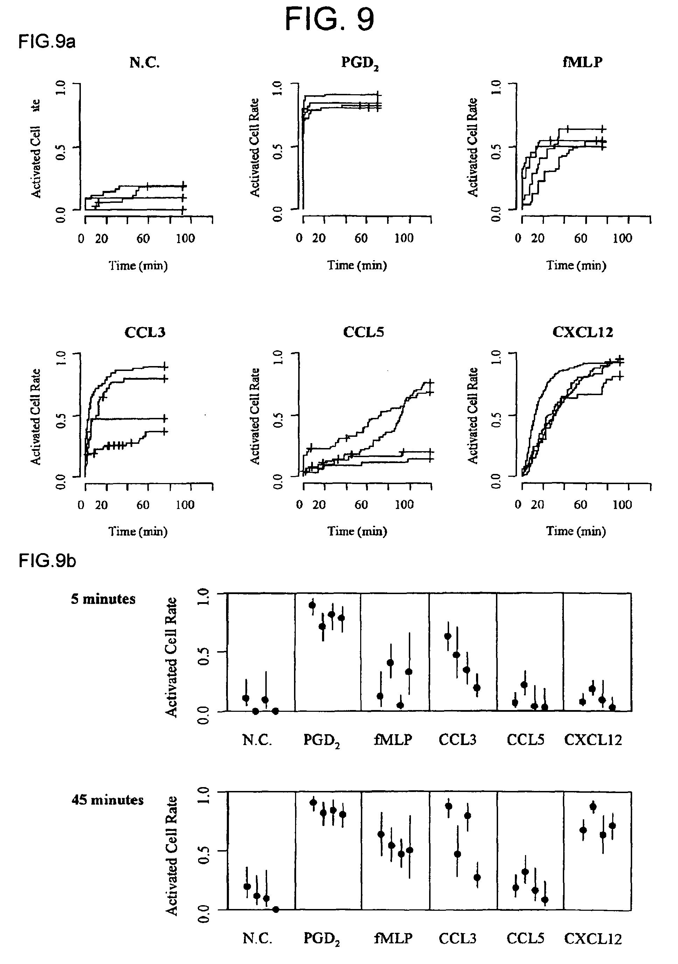

In the VD plot, median values were calculated from the time-series data of the velocity and the directionality, and the data was plotted. Therefore, even if there was a cellular reaction against the chemotactic factor, this reaction might not be detected in the VD plot, in the case where the motility state continued for a time period relatively shorter than the period of static state. Here, in order to check presence or absence of cellular reaction against the chemotactic factor and also to know the time taken by the reaction, Chemotactic Response Time (CRT) Analysis was conducted. The CRT indicated a response time taken from the time when the chemotactic factor was injected until the time when the cell started migration (FIG. 6d), and according to the CRT distribution of the cells under each experimental condition, it was possible to evaluate a cellular reactivity against the chemotactic factor. In this example, the CRT was calculated for each cell from the time-series data of the cell velocity, and based on the calculated data, there was also calculated a time series variation of the rate of cells which started movement (Activated Cell Rate: ACR) (FIG. 9a).

In calculating the CRT, since the cell velocity hardly exceeded 0.15 µm/min in the sample without injection of chemotactic factor (FIG. 7 and FIG. 8a), the value 0.15 µm/min was used as a threshold for detecting the cell migration.

FIG. 9b illustrates estimate values and 95% confidence interval of the ACR as to each sample, after lapse of 5 minutes and 45 minutes from the start of reaction. According to FIG. 9a, it was found that the ACR remained unchanged around 0 to 0.2, in the case of N.C., and almost no cells started moving, whereas under the concentration gradient condition of chemotactic factors there was shown a state where the migration was started gradually. In the PGD₂ treatment, the cell started migration within an extremely short period of time compared to the other factors, and according to the graphs of FIG. 9a and FIG. 9b, it was found that around 80% of the cells started moving within around 5 minutes after the measurement was started. As for fMLP, CCL3, and CXCL12, the cells started moving gradually as time advanced, but as for CCL3, there were outstanding variations among the samples. In addition, as for CCL5, the cells hardly started migration after the lapse of 5 minutes and 45 minutes from the measuring start, but it was observed that CCL5 had a characteristic tendency, namely, the cellular reactivity gradually turned up later on.

### (Lifetime analysis of cellular chemotaxis)

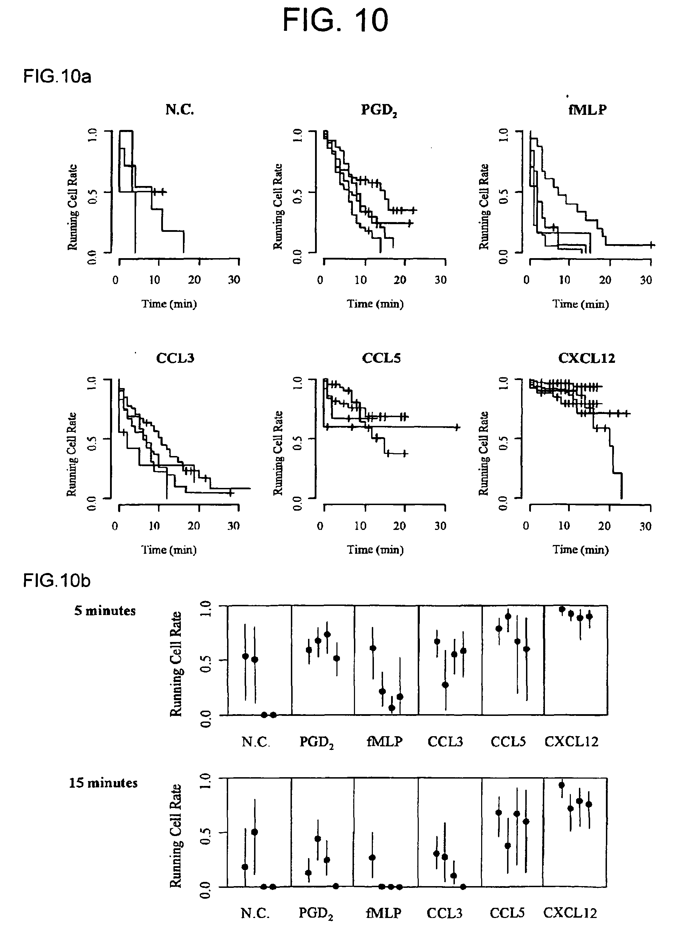

Elapsed time from when the eosinophil started migration until the migration stop (Chemotactic Lifetime: CLT) was analyzed. In calculating the CLT, similar to the calculation of CRT, the state where an EMA value of the velocity exceeded 0.15 µm/sec was defined as the cell was migrating. FIG. 10a illustrates a result of calculating a rate of still migrating cells (Running Cell Rate: RCR), every elapsed time after the start of movement, according to the CLT of individual cells. FIG. 10b illustrates the values of RCR and 95% confidence intervals after lapse of 5 minutes and 15 minutes from the start of movement. It was found that as for PGD₂, fMLP, and CCL3, less than around 50% of the cells continued migration for 15 minutes or longer, and in particular, the migration of eosinophils toward CCL5 and CXCL12 had a long lifetime tendency, compared to the case of fMLP, as to which most of the cells stopped migration within 15 minutes.

### (Chemotaxis analysis of eosinophil of allergic patient and asthmatic patient)

It is known that eosinophil relates to various clinical conditions, including allergic disease. The use of TAXIScan allows a detailed analysis of the activity of eosinophil from the viewpoint of chemotaxis. Therefore, the eosinophil chemotaxis was compared among each of the various disease groups, and a biomarker extraction was studied, which was envisioned to be applied to a diagnosis technique.

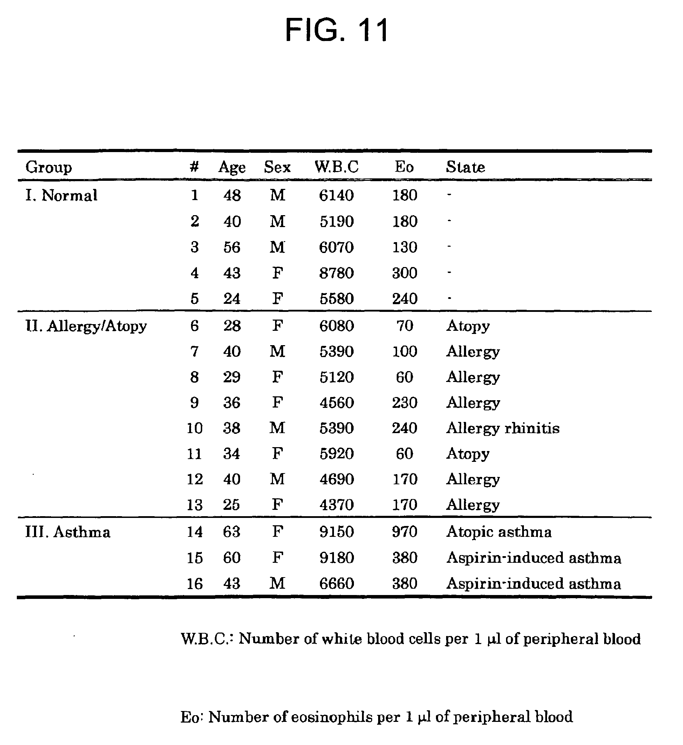

Eosinophils obtained from normal healthy donors, allergy patient donors, atopy patient donors and asthma patient donors were used, and a chemotactic assay according to TAXIScan was conducted. FIG. 11 illustrates donor information of the blood samples that were used for the analysis. Here, the samples were categorized into three groups; normal subject (I), allergy or atopy patient (II), and asthma patient (III). The number of the blood samples used for the analysis was; five for group I, eight for group II, and three for group III.

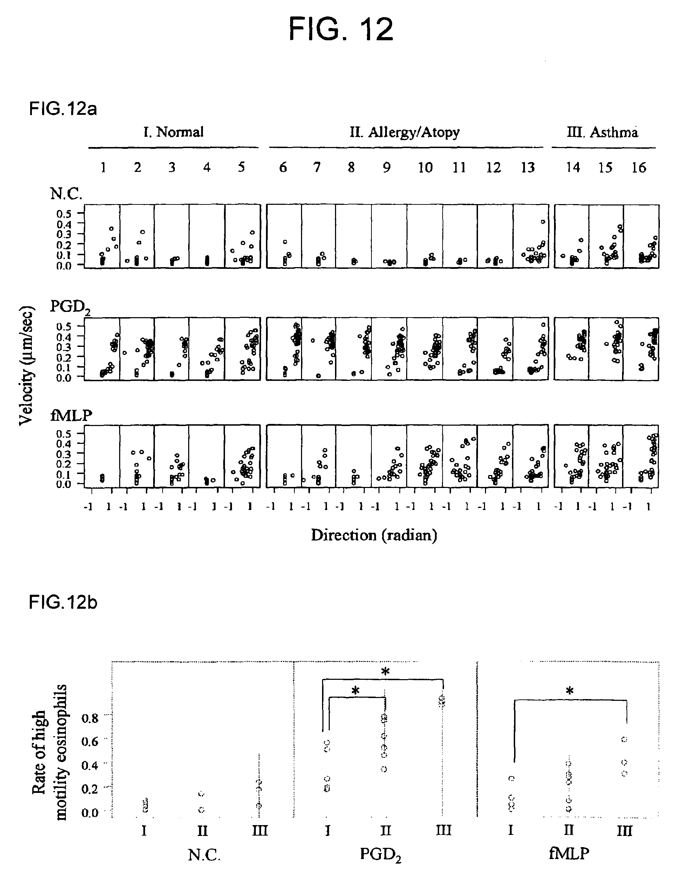

The eosinophil chemotaxis assay was carried out as to the samples to which any concentration gradient of chemotactic factors was not added (N.C.), and samples having the concentration gradient conditions of PGD₂ and fMLP. FIG. 12a and FIG. 12b illustrate the result of the assay using the VD plot. When the cell motility obtained from the VD plot was compared among the disease groups, there were recognized a significant difference in the asthma patients (III) when the fMLP stimulus was used, and a significant difference in the allergy/atopy patients (II) and the asthma patients (III) when the PGD₂ stimulus was used, compared to the normal subject (I). In addition, it was found that the cell motility of eosinophil toward these chemotactic factors were high in the inflammatory diseases (FIG. 12b).

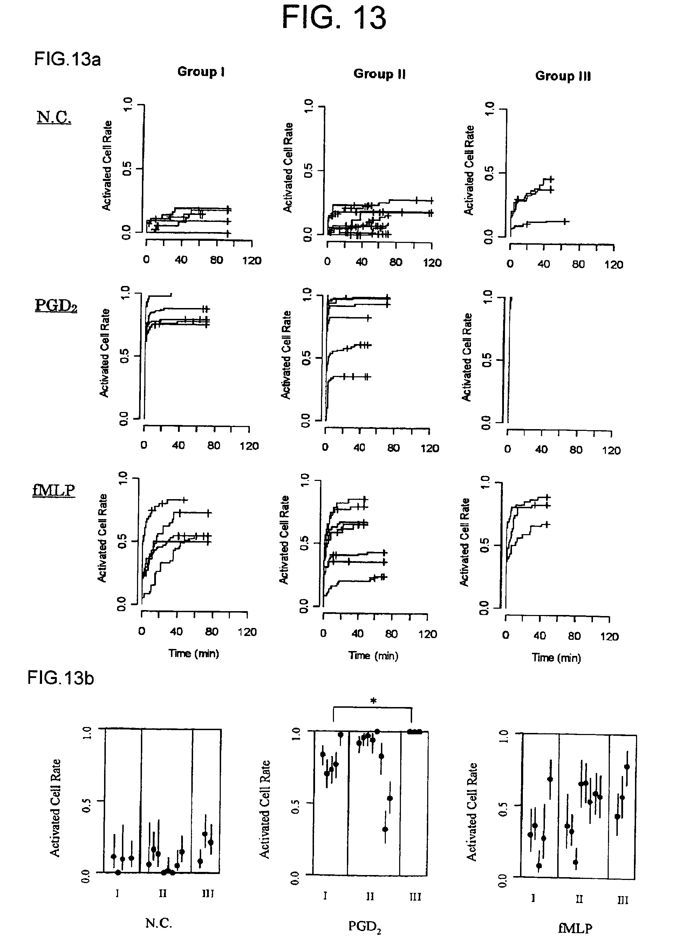

FIG. 13a and FIG. 13b illustrate the results of the CRT analysis that was carried out for the eosinophils as to each of the samples. The asthma patients (III) showed an extremely high reactivity toward PGD₂, with a result that all the eosinophils started migration within 5 minutes, and there was a significant difference of 5%, from the normal subject (I). However, there was not found a significant difference among the groups as to the other chemotactic factors.

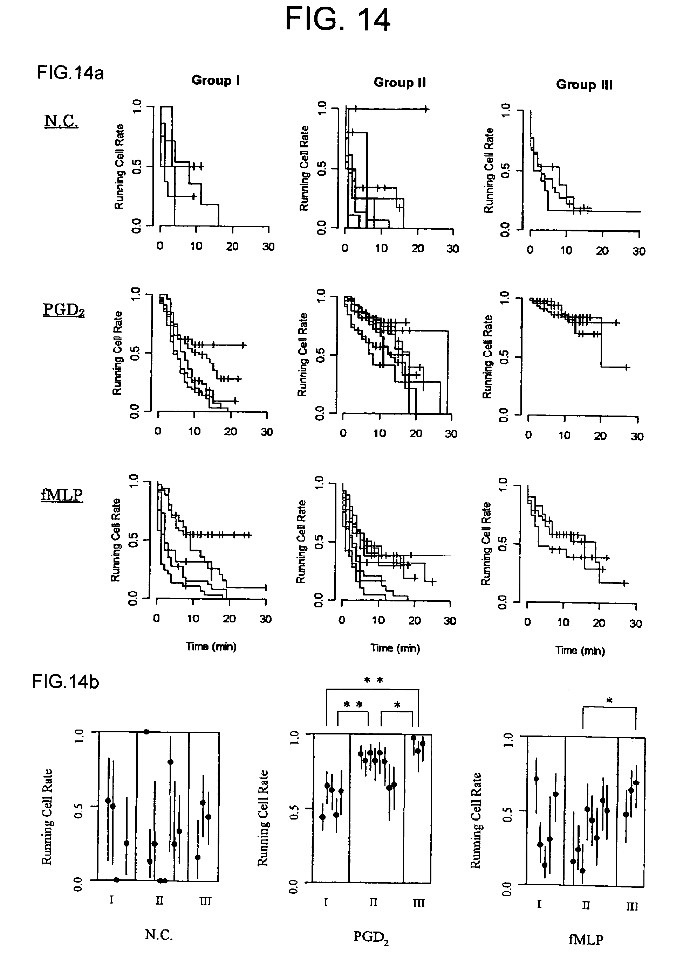

Subsequently, the CLT Analysis was carried out in order to compare the duration time of the cellular migration among the patient groups. It was clarified that when stimulated by the PGD₂ concentration gradient, the duration time of the cellular migration was long in the groups of allergy/atopy patients and asthma patients, and the rate of the cells that continued migration for five minutes or more was significantly higher, compared to the normal subjects (FIG. 14a and FIG. 14b). On the other hand, in the case of fMLP, there was not found a significant difference between the normal subjects and the patients of inflammatory diseases.

In the present study, a novel technique for quantifying the cellular chemotaxis has been established, and the effect thereof has been verified by conducting the chemotactic analysis of eosinophil.

The eosinophil is a kind of leucocyte originated from bone marrow, and most eosinophils exist within tissues, serving as a biological defense against a foreign body such as a parasite that entered through the epithelium. It is known that proliferation and differentiation of the eosinophils are stimulated in the bone marrow by cytokine stimuluss such as IL-3, IL-5, and GM-CSF, and IL-5 controls mobilization of eosinophils into the peripheral blood (Collins et al., 1995, Lampinen et al., 2004). Most of the eosinophils circulating through the blood infiltrate the tissues, and after activated therein, inducing Fc epsion RI to appear on the cell surface, and a biological defense reaction is shown against the parasite mediated by antibody (Gounni et al., 1994). It is considered that the mobilization and activation of the eosinophils toward an infected area are triggered mainly by degranulation of mast cell and activation of T_{H}2 cell.

It is further considered that the eosinophil chemotaxis is extremely important to present such physiological cell activities as described above. As a response of eosinophil to the chemotactic factors, there are reported as the following; a rise of cAMP, activation of actin polymerization, activation of adhesion factor, and the like (Monneret et al., 2001). Each of such activities has a significant meaning, and by employing the chemotactic analyzing method newly developed here, it is now possible to check a reaction of eosinophil against ligand, from various approaches.

The state of eosinophil motility toward various chemotactic factors was analyzed by using the chemotaxis analysis technique newly developed in the present study, and there have been clarified various patterns depending on the factors. In the case of PGD₂, the time taken by the cells to start moving was extremely short compared to other chemotactic factors, and after lapse of 5 minutes from the assay start, about 70% to 100% of the cells started movement. In the case of fMLP, about half of the eosinophils migrated in the normal subjects according to the CRT analysis, but migration duration time was relatively shorter than the other factors. As for chemokines, three types; CCL3, CCL5, and CXCL12 were analyzed. According to the CRT Analysis, there was found a characteristic state that in CCL5 treatment, the cellular reactivity turned up gradually after lapse of approximately one hour from the reaction start. A difference in the rate of diffusion velocity of the chemotactic factors may cause a variation of the reaction response time. However, since any of the CCL3, CCL5, and CXCL12 are chemokines having a similar structure, it is hard to assume that there is a large difference in a physical diffusion. Therefore, it is considered that there is a difference in reactivity of the cell itself against a factor. In addition, according to the CLT Analysis, as for the CCL3, it was found that the migration duration time was short relative to CCL5 or CXCL12. It is interesting that even if an identical eosinophils are used, different chemotactic patterns are appeared depending on the chemotactic factor being given. The reason for that can be assumed that there exist a difference in a binding phase between the chemotactic factor and a receptor, and/or a difference in a signal transduction system in the downstream. It is extremely important to clarify the mechanism which causes such differences above and to study its physiological significance. Moreover, further development of the study can be expected by applying the analysis method established here.

The eosinophil chemotaxis is very interesting also from a pathological point of view. The eosinophil is partially responsible for a biological defense reaction, but it is also known that the number of eosinophils specifically increases at an inflammatory site of a disease such as asthma. It is considered that abnormal functioning of the eosinophil aggressivity induces inflammatory diseases such as asthma, allergic rhinitis, and atopic dermatitis. In the various inflammatory diseases, proliferation and differentiation of the eosinophils are activated in the bone marrow by the cytokine stimulus such as IL-5, and further, the eosinophils in the blood are also subjected to priming and then the cellular mechanism is activated as a whole. It is considered that the mechanism for mobilizing the activated eosinophils toward the inflammatory site is related to multiple factors. Firstly, the eosinophils and adhesive molecules of vascular endothelial cells are activated by the cytokine actions. Therefore, rolling and adhesion of eosinophils are increased in the vascular endothelium. Secondly, the activated eosinophils have a high ability to autonomously infiltrate into the tissue, passing through the cell wall. Therefore, in the allergy and asthma patients, migration of eosinophils into the tissue is increased. Thirdly, since the activated eosinophils enhance migration potency toward various chemotactic factors, the migration toward the inflamed area focus within the tissue becomes active. According to the multifaceted actions, it is considered that the eosinophils are accumulated on the inflammatory site in the inflammatory diseases such as allergy and asthma (Wardlaw, 2001, Lampinen et al., 2004).

In the present study, eosinophil chemotaxis patterns were compared among the normal subjects and inflammatory disease patients, such as allergy and asthma. As a result of comparison of eosinophil chemotaxis toward PGD₂, it was found that the eosinophils originating from allergic/atopic patients and asthmatic patients showed a high motility, according to the VD plot analysis. According to the CRT Analysis, it was clarified that the asthmatic patients had high reactivity in starting movement compared to the normal subjects, and further the CLT (Chemotactic Lifetime) showed a distinctive feature. In other words, the rate of cells that moved continuously for five minutes or longer from the migration start was particularly high in the group of allergic/atopic patients, compared to the normal subject group, and the asthmatic patient group showed a much higher rate. On the other hand, in the experiment using fMLP, a particularly higher movement activity was found in the asthmatic patients than the normal subjects according to the VD plot analysis, but as for the CRT and CLT, there was not a large difference between the normal subjects and the patients. The CLT of the eosinophil chemotaxis toward PGD₂ was different depending on the disease groups, but in the migration experiment toward fMLP, simultaneously conducted, there was not such distinctive CLT difference. Therefore, the results above cannot be explained by the reasons such as machinery related to the cell motility or consumable energy for the cell motility. Accordingly, it may be hypothesized that , for example, in the patient of inflammatory disease, any mechanism for controlling excessive chemotaxis did not work properly.

According to the quantification technique of cellular chemotaxis, being established in the present invention, multifaceted analysis of the cellular chemotaxis is now enabled, which has been impossible conventionally. According to the assay using the eosinophil, it is shown that the method currently established is useful also as a technique for evaluating the diseases such as allergy, in addition to basic studies of the cellular chemotaxis. In addition to eosinophil, various cells such as neutrophil, basophil, lymphocyte, and monocyte are used as the cell which shows the chemotaxis. It is further expected that the present technique is effective as a tool to conduct diagnosis and evaluation of a disease to which the cellular chemotaxis is related. Even more particularly, the achievement of the present invention gives indications about a mechanism in which inflammation occurs and gets worse in the patient of a disease such as allergy and asthma. It is understood that elucidation of the mechanism will be linked to a promising target for the inflammatory diseases, and further development of a new drug will be conducted effectively by using the method established by the present invention. Therefore, further development of the study can be expected.

## Claims

1. A cell motility measuring method for measuring a cell motility state, comprising the steps of;
placing living cells in an observation space in such a manner as movable on a plane,
injecting a stimulus that exerts an effect on the cell motility into the observation space in which the cells are accommodated, and
measuring at least either one of the factors;
(a) a reaction time, as to each of the cells, required from a stimulus injection time point to a time of starting movement, and
(b) a duration time while the movement continues, as to each of the cells that started the movement.

2. The cell motility measuring method according to claim 1, wherein,
in the factor (a), the time of starting movement is defined as when the cell motility reaches at least a threshold that is predetermined.

3. The cell motility measuring method according to claim 2, wherein,
measuring the reaction time includes,
time-course imaging of the observation space where the cells exist by an imaging device at multiple imaging time points,
storing images acquired by the imaging device in a storage device via a computer, and records the multiple imaging time points respectively in association with the images being acquired, and
the computer performs,
a process for extracting from the images being acquired, a distance between a position of each of the cells shown in the image being acquired at each imaging time point, and another position shown in the image of each of the cells taken at the next time point,
calculating an elapsed time between the imaging time points to reach the distance, and calculating a velocity of each of the cells by dividing the distance by the elapsed time,
determining whether or not the velocity being calculated at each time point for each of the cells reaches at least the threshold, and at the time point when reaching at least the threshold, calculating a time required from the stimulus injection time point to the time point reaching at least the threshold, so as to obtain the reaction time.

4. The cell motility measuring method according to claim 1, wherein,
the duration time being measured in the item (b) is defined as a time taken from the time when reaching at least a predetermined threshold, up to the time when reaching lower than the predetermined threshold.

5. The cell motility measuring method according to claim 4, wherein,
measuring the duration time includes,
time-course imaging of the observation space where the cells exist by an imaging device at multiple imaging time points,
storing images acquired by the imaging device in a storage device via a computer, and records the multiple imaging time points respectively in association with the images being acquired, and
the computer performs,
a process for extracting from the images being acquired, a distance between a position of each of the cells shown in the image being acquired at each imaging time point, and another position shown in the image of each of the cells taken at the next time point,
calculating an elapsed time between the imaging time points used to reach the distance, and calculating a velocity of each of the cells by dividing the distance by the elapsed time,
determining whether or not the velocity being calculated at each time point for each of the cells reaches at least the threshold, determining whether or not the velocity being calculated reaches lower than a predetermined threshold, and calculating a time required from the time point reaching at least the threshold up to the time point reaching lower than the predetermined threshold, so as to obtain the duration time.

6. The cell motility measuring method according to any of claims 1 to 5, wherein,
the cells being used are selected from eosinophil, neutrophil, basophil, monocyte, T-lymphocyte, B-lymphocyte, macrophage, mast cell, and dendritic cell.

7. A diagnosis support method using the cell motility measuring method according to any one of claims 1 to 5, wherein,
eosinophils are used as the cells, prostaglandin is used as the stimulus, and a length of the duration time concerning eosinophils being measuring targets is used as an index for diagnosing allergy, atopy, or asthma.

8. A cell motility measuring system for measuring a cell motility state, comprising,
an observation support unit which constitutes an observation space for holding living cells in such a manner as being movable on a plane, and which is capable of injecting into the observation space, a stimulus for exerting an effect on the cell motility,
an imaging device for taking images of the cells within the observation support unit, and
a computer for processing the images acquired by the imaging device, wherein,
the computer is connected to a storage device, a display device, and an input device, and comprising
a means for storing the images from the imaging device in the storage device and stores time information indicating imaging time points, respectively in association with the images,
a means for accepting of an input of stimulus injection time point via the input device, acquiring and storing the time point in the storage device, and
a means for obtaining an evaluation index of the cell motility by analyzing the images being acquired, wherein,
the means for obtaining the evaluation index executes the processing of,
extracting from the images being acquired, a distance between a position of each of the cells shown in the image acquired at each imaging time point, and a position of each of the cells shown in the image acquired at the next imaging time point,
calculating an elapsed time between the imaging time points used for obtaining the distance, and calculating a velocity of each of the cells by dividing the distance by the elapsed time, and
determining whether or not the velocity being calculated for each time point with respect to each of the cells reaches at least a threshold, and calculating a time required from the stimulus injection time point up to the time when reaching at least the threshold, so as to obtain a reaction time.

9. A cell motility measuring system for measuring a cell motility state, comprising,
an observation support unit which constitutes an observation space for holding living cells in such a manner as being movable on a plane, and which is capable of injecting into the observation space, a stimulus for exerting an effect on the cell motility,
an imaging device for taking images of the cells within the observation support unit, and
a computer for processing the images acquired by the imaging device, wherein,
the computer is connected to a storage device, a display device, and an input device, and comprising
a means for storing the images from the imaging device in the storage device and stores time information indicating imaging time points, respectively in association with the images,
a means for accepting of an input of stimulus injection time point, acquiring and storing the time point in the storage device, and
a means for obtaining an evaluation index of the cell motility by analyzing the images being acquired, wherein,
the means for obtaining the evaluation index executes the processing of,
extracting from the images being acquired, a distance between a position of each of the cells shown in the image acquired at each imaging time point, and a position of each of the cells shown in the image acquired at the next imaging time point,
calculating an elapsed time between the imaging time points used for obtaining the distance, and calculating a velocity of each of the cells by dividing the distance by the elapsed time, and
determining whether or not the velocity being calculated for each time point with respect to each of the cells reaches at least a threshold, determining whether or not the velocity being calculated for each time point with respect to each of the cells reaches lower than the threshold, and calculating an elapsed time between the time point reaching at least the threshold and the time point reaching lower than the threshold, so as to obtain a duration time.

10. The cell motility measuring system according to claim 8, further executes a processing of,
determining whether or not the velocity being calculated for each time point with respect to each of the cells reaches at least the threshold, determining whether or not the velocity being calculated for each time point with respect to each of the cells reaches lower than the threshold, calculating an elapsed time between the time point reaching at least the threshold and the time point reaching lower than the threshold, so as to obtain a duration time.
